# EUROPEAN PATENT APPLICATION

(11) **EP 1 038 532 A1**
(43) Date of publication of application: **27.09.2000**
(21) Application number: 98920755.0
(22) Date of filing: 09.04.1998
(51) Int. Cl.: A61K 38/14, A23L 1/30

(54) **IMMUNOMODULATOR AND PHARMACEUTICAL COMPOSITIONS WITH ANTITUMOR PROPERTIES, AND A FOOD ADDITIVE**

(30) Priority: 11.04.1997 RU 97105213; 11.04.1997 RU 97106270
(71) Applicant: Kaljuzhin, Oleg Vitallievich, Moscow, 117418 (RU); Shkalev, Mikhail Vladimirovich, Moscow, 117418 (RU)
(72) Inventor: Kaljuzhin, Oleg Vitallievich, Moscow, 117418 (RU); Shkalev, Mikhail Vladimirovich, Moscow, 117418 (RU)
(74) Representative: Gerbino, Angelo
(86) International application number: RU9800102
(87) International publication number: WO9846251

(57) **Abstract**

The invention proposes the use of α and/or β-glycosides N-acetylmuramil-L-alanyl-D-isoglutamine /CH₃-/CH₂/ₙ-muramildipeptide (MDP) as an immunomodulator, as well as pharmaceutical compositions with antitumor properties that include an effective quantity of α and/or β-glycosides muramildipeptide (MDP) and physiologically acceptable carriers. The dosage of the active ingredient in the form of a drug is of 0.0001-100 mg/kg in weight when administered perorally, of 0.00001-30 mg/kg in weight when administered intravenously, and of 0.00001-10 mg/kg in weight in the case of a lyposomal administration. This invention also relates to a preparation formulated on the basis of α and/or β-glycosides MDP/CH₃-/CH₂/ₙ-MDP/ which activates the main immunity bonds. The invention further proposes the use of α and/or β-glycosides N-acetylmuramil-L-alanyl-D-isoglutamine as a food additive.

## Description

### Sphere of technique.

The invention concerns medicine and in particular preparations, which are used to correct the immunity of the organism and represent highly effective immunomodulators. The invention also concerns sphere of food preparation and specifically food additives with prophylactic and treating activities.

### Previous level.

At present, great attention is paid to reactivation of natural defense reactions of the body against tumors as one of the important tasks of complex therapy in neoplastic diseases. For this purpose immunomodulators, which are able to increase antitumor immune response, are used. N-acetylmuramyl-L-alanyl-D-isoglutamine (muramyl dipeptide, MDP) ranks high among great number of endogenic and exogenic immunomodulators.

Muramyl peptide is known to have stimulatory effect on the main components of the immune system. However, after systemic administration it is rapidly cleared from the body due to high hydrophilicity. Moreover, it has a significant concomitant pyrogenic effect. As a rule, lipophilic derivatives of MDP are characterized by a more effective stimulation of tumoristatic and tumoricidal activities of macrophages, a more prolonged delay in the body, and a lower pyrogenicity in comparison with hydrophilic analogues.

Glycosides of N-acetylmuramyl-L-alanyl-D-isoglutamine /CH₃-(CH₂)n-MDP/ are described in scientific and technical literature [Zemlyakov A.E. & Chirva V.Ya. XV Ukrainskaya respublikanskaya po organicheskoi khimii. Tezisy doklada. Uzhgorod, 1986, p.409.].

Some of food additives, which are introduced into food, are known to have treating and prophylactic effects on various functions of the human body. Among them there are derivatives of peptides: β-lactoglobulin, proglamine, α-poly-L-lysine, etc. [see EP No. 0540462, cl. A23L1/305, 1990; DE No. 4040874, cl. A23L1/03, 1990].

### Description of invention.

The invention is to solve the task of creation of substances, which represent immunomodulators and are effective for correction of the immunity in cases of serious immune insufficiency. The other purpose of the invention is to create a new food additive, which, when introduced into food, acts as an immunomodulator and antitumor remedy.

To solve these problemes, a group of inventions united by mutual inventor's intention is proposed. Inventors propose the use of α- and/or β-glycosides of N-acetylmuramyl-L-alanyl-D-isoglutamine /CH₃-(CH₂)n-MDP/ as an immunomodulator. On basis of this immunomodulator, inventors further propose pharmaceutical composition with antitumor properties that include an effective quantity of α- and/or β-glycosides of N-acetylmuramyl-L-alanyl-D-isoglutamine /CH₃-(CH₂)n-MDP/ and physiologically acceptable carriers.

Pharmaceutical composition contains the active ingredient in dose of 0.00001-30 mg/kg in weight when administered intravenously, subcutaneously and intramuscularly, of 0.0001-100 mg/kg when administered perorally, and of 0.0001-10 mg/kg in weight in case of a lyposomal administration.

α- and/or β-glycosides of N-acetylmuramyl-L-alanyl-D-isoglutamine are also used as a food additive.

Abbreviations of the active compounds: α- and/or β-glycosides of MDP, or CH3-(CH2)n-MDP.

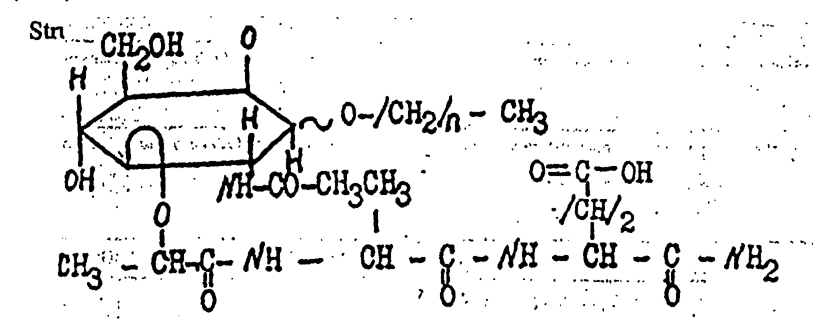

Experimental studies have shown that amphiphilic preparation β-heptylglycoside-MDP (C₇H₁₅MDP) is a more effective stimulator of main components of the immunity in comparison with MDP and number of its derivatives in particular lipophilic homologue β-hexadecylglycoside-MDP (C₁₆H₃₃MDP). Advantages of amphiphilic preparation in penetration through biological membranes and peculiarities of intracellular traffic explain above-mentioned distinctions of the MDP derivatives. Positive results of investigation of C₇H₁₅MDP biological activities as stimulator of antitumor response in mouse melanoma in lymphoma models as well as low toxicity point to the expediency of clinical use of this MDP derivative. Amphiphilicity is an important property of MDP derivatives, which allows them to penetrate actively through biological membranes and influence cytoplasmatic processes leading to lymphocyte and macrophage activation. Amphiphilic preparations have advantages in comparison with well-known lipophilic derivatives, such as muramyl tripeptide-phosphatidylethanolamine (MTP-PE), MDP-glyceroldipalmitate (MDP-GDP), muramyl tripeptide-cholesterol (MTP-Chol), B-30-MDP, in terms of better solubility in water solutions, which expands the forms of their applications. Moreover, due to definite hydrolipophilic balance, amphiphilic preparations can be effectively encapsulated into different vehicles, such as liposomes, being localized both in aqueous and lipid phases of liposomes. Besides, these preparations are characterized by more prolonged delay in the body in comparison with MDP and its hydrophilic analogues.

### Study of MDP glycoside biological activities.

### 1. Immunomodulatory activities of MDP derivatives.

C₇H₁₅MDP, C₁₆H₃₃MDP and MDP were compared in all functional tests in vitro in equimolar concentrations.

**Table 1**

| The influence of MDP and its derivatives on proliferation of lymphocytes in a mixed lymphocyte culture (MLC). | | | |
|---|---|---|---|
| | Concentrations of MDP and its derivatives (µM) | | |
| | 20 | 10 | 5 |
| Control | 11.9±0.9 | | |
| MDP | 14.6±1.0 | 14.1±0.8 | 12.7±1.2 |
| C₇H₁₅MDP | 15.9±1.4 | 16.4±0.6 | 14.5±0.9 |
| C₁₆H₃₃MDP | 5.9±0.8 | 7.7±0.4 | 9.5±1.2 |

Each value in the table is the mean index of proliferation stimulation of lymphocytes in MLC ± S.D. of six independent experiments.

Amphiphilic preparation β-heptylglycoside-MDP has been shown to have more significant stimulatory effect on main populations of cells-effectors of antitumor response compared to unmodified MDP and lipophilic analogue β-hexadecylglycoside-MDP. C₇H₁₅MDP has been found to stimulate lymphocyte proliferation in mixed lymphocyte culture, to increase cytotoxicity of natural killers (NK), to activate allospecific cytotoxic T-lymphocytes (CTTL) generation and interleukin-1 (IL-1) and tumor necrosis factor (TNF) production by macrophages.

### 2. The interaction of the MDP derivatives with model membranes.

In order to explain distinctions in immunomodulatory activities of amphiphilic and lipophilic homologues, the interaction of these muramylpeptides with several model membranes.

Hydrolipophilic balance of the preparations can significantly influence the interaction of homologous MDP derivatives with cell membranes and their internalization into cells and intracellular traffic. To demonstrate the differences in hydrolipophilic balance of two homologues, the simplest model water/chloroform "membrane" have been used.

Marked differences in distribution of ₁₄C-labelled preparations in the phases of this model "membrane" have been found. 64% of C₇H₁₅MDP was included in the water phase, and 36% of the preparation in the organic phase. At the same time, the water phase included only 28% of its lipophilic homologue, and the organic phase 72%.

The other model lipid membrane prepared by means of dipping of filter discs in equimolar mixture of cholesterol and phosphatidylcholine in bensol have been used to study kinetics of the MDP derivatives from one water phase to the other and delay of the preparations in lipid barrier.

Preparation C₁₆H₃₃MDP appeared in trace quantity in the second water phase in 1 h, and after 6 h of experiment its concentration in this phase became significant. But its further penetration through lipid barrier was significantly slower in comparison with C₇H₁₅MDP. The former appeared in the second water phases only in 24 h. However, speed of its penetration then increased and after 72 h C₇H₁₅MDP was thus the first to reach a state of equilibrium between the preparation concentrations in water phases from both sides of the model membrane.

**Table 2**

| LPS-induced proliferation of mouse splenocytes under the influence of MDP and its derivatives. | | | |
|---|---|---|---|
| | Concentrations of MDP and its derivatives (µM) | | |
| | 20 | 10 | 5 |
| Control | 3.4±0.2 | | |
| MDP | 4.1±0.5 | 4.4±0.1 | 4.1±0.4 |
| C₇H₁₅MDP | 4.4±0.3 | 4.6±0.3 | 4.4±0.3 |
| C₁₆H₃₃MDP | 3.0±1.0 | 2.6±0.3 | 3.0±0.1 |

Each value in the table is the index of proliferation of lymphocytes in response to stimulation of 1 µg/ml under the influence of MDP derivatives (arithmetical mean ± S.D. of three independent experiments).

**Table 3**

| The influence of MDP and its derivatives on the generation of allospecific cytotoxic T lymphocytes (CTLs) in a mixed lymphocyte culture. | | | | |
|---|---|---|---|---|
| Concentrations of MDP and its analogues (µM) | | Effector:target cells | | |
| | | 30:1 | 10:1 | 3:1 |
| | Control | 71±5 | 51±3 | 22±4 |
| 20 | MDP | 82±3 | 60±2 | 31±3 |
| | C₇H₁₅MDP | 94±4 | 73±4 | 46±6 |
| | C₁₆H₃₃MDP | 35±17 | 23±6 | 9±3 |
| 10 | MDP | 81±2 | 64±5 | 30±6 |
| | C₇H₁₅MDP | 87±6 | 74±4 | 36±6 |
| | C₁₆H₃₃MDP | 52±13 | 27±6 | 11±9 |
| 5 | MDP | 77±7 | 58±7 | 27±3 |
| | C₇H₁₅MDP | 83±5 | 62±4 | 35±7 |
| | C₁₆H₃₃MDP | 69±10 | 42±7 | 19±1 |

Each value in the table is the cytotoxic activity of CTLs (%) with respect to allogenic tumor cells P815 (arithmetical mean ± S.D. of three independent experiments).

Detection of membrane-associated radioactivity after the end of the experiment revealed that C₇H₁₅MDP bound to the model membrane four times less intensively than its lipophilic analogue.

To understand better how the immunomodulators-derivatives of MDP interact with biological membranes and penetrate into cells, the uptake of the ₁₄C-labelled immunomodulators (C₇H₁₅MDP and C₁₆H₃₃MDP) by human erythroleukemia K562 cells have been investigated.

Although the uptake of immunomodulators by cells depends not only on the physicochemical properties of the preparation but also on some other factors, the results of this set of experiments correlated in general with the data on the interaction of the MDP derivatives with the model cholesterol/phospholipid membrane.

One hour after the start of cell cultivation in a medium lacking glutamine (for suppression of cell division) and containing radiolabelled MDP analogues, the lipophilic derivative C₁₆H₃₃MDP bound to the cells 50% more intensively than its amphiphilic homologue (p<0.05). However, C₁₆H₃₃MDP uptake then slowed down and after 2 h of incubation the cell-associated radioactivity practically entered the "plateau" stage. C₇H₁₅MDP, which bound to the cells not so intensively at the beginning of the experiment, did not enter the phase of equilibrium between the uptake and efflux after 2-4 h. As a result, in 24 h the uptake of C₇H₁₅MDP by cells exceeded by 25% the uptake of its more lipophilic analogue (p<0.05).

Thus, significant quantity of C₁₆H₃₃MDP delayed in the plasma membrane. Of course, lipophilic preparation associated with plasma membrane is to be internalized actively by cells as a result of endocytosis. However, the exocytosis evidently soon balances this process.

On the contrary, amphiphilic C₇H₁₅MDP, which was included relatively slowly into the membranes at the beginning, then left easily, which finally gave it advantages in penetration through lipid membrane and in the uptake by cells, compared to the more lipophilic C₁₆H₃₃MDP. Amphiphilicity of the preparation C₇H₁₅MDP is a principal character that allows effective penetrating through biological membranes, which correlates with marked activation of the main cells-effectors of antitumor immune response.

**Table 4**

| Production of IL-1 (a) and TNF (b) by C57BL/6 mouse peritoneal macrophages cultured in presence of MDP and its derivatives. | | | |
|---|---|---|---|
| | Concentrations of MDP and its analogues (µM) | | |
| | 20 | 10 | 5 |
| | | | |

| (a) Proliferation of tymocytes cultured with samples of IL-1 (index of proliferation) | | | |
|---|---|---|---|
| Control | 1.3±0.4 | | |
| MDP | 3.9±0.3 | 3.8±0.3 | 3.4±0.2 |
| C₇H₁₅MDP | 4.8±0.2 | 3.9±0.2 | 3.8±0.2 |
| C₁₆H₃₃MDP | 3.9±0.1 | 3.9±0.4 | 3.8±0.1 |

| (b) Cytosis of L929 cells by samples of TNF (%) | | | |
|---|---|---|---|
| Control | 7±6 | | |
| MDP | 45±4 | 45±3 | 40±3 |
| C₇H₁₅MDP | 62±5 | 57±8 | 41±2 |
| C₁₆H₃₃MDP | 47±10 | 40±16 | 36±15 |

Each value in the table is the mean ± S.D. of three independent experiments.

**Table 5**

| The influence of MDP and its derivatives (10 µM) on cytotoxic activity of natural killers (NK) with respect to NK-sensitive lymphoma YAC-1 cells. Effector:target cell ratio is 25. | |
|---|---|
| MDP and its derivatives | Cytotoxicity (%) |
| Control | 24±2 |
| MDP | 28±2 |
| C₇H₁₅MDP | 31±2 |
| C₁₆H₃₃MDP | 21±3 |

Each value in the table is mean index of cytotoxicity ± S.D. of three independent experiments.

### 3. Antitumor activities of C₇H₁₅MDP in mouse lymphoma and melanoma models.

*Experimental procedures*. Briefly, suspension of lymphoma EL-4 or melanoma B16 cells (10⁶ cells) was injected subcutaneously into the footpad of C57BL/6 mice. Then mice underwent course of C₇H₁₅MDP treatment. Solution of the preparation in phosphate buffer saline (PBS) was administered i.v. or p.o. Single dosage for p.o. administration was of 1 mg/kg in weight, and of 0.5 mg/kg in weight when administered i.v. The preparation was administered twice a week during 3 weeks, beginning immediately after implantation of tumor cells. Besides, some tumor-bearing mice were treated with the preparation encapsulated in liposomes consisting of phosphatidylcholine:phosphatudylserine mixture (7:3).

Liposomal C₇H₁₅MDP in dose of 0.25 mg/kg was administered i.v. by the same schedule that was used with unencapsulated preparation.

Control mice were treated with PBS without active compound.

Antitumor effect was estimated using the following criteria: (1) prolongation of life-span, (2) inhibition of tumor growth.

**Table 6**

| The influence of C₇H₁₅MDP on life-span of tumor-bearing mice. | | | |
|---|---|---|---|
| Tumor | Treatment | Mean life-span (days) | Prolongation of life-span (%) |
| | | | |

| (a) Melanoma B16 | | | |
|---|---|---|---|
| | Control (PBS p.o.) | 30±4 | - |
| | Control (PBS i.v.) | 36±3 | - |
| | C₇H₁₅MDP p.o. | 51±5 | 70 |
| | C₇H₁₅MDP i.v. | 74±9 | 101 |

| (b) Lymphoma EL-4 | | | |
|---|---|---|---|
| | Control (PBS p.o.) | 42±6 | - |
| | Control (PBS i.v.) | 39±4 | - |
| | C₇H₁₅MDP p.o. | 50±7 | 19 |
| | C₇H₁₅MDP i.v. | 63±5 | 62 |

**Table 7**

| Inhibition of tumor growgth under the influence of C₇H₁₅MDP. | | | |
|---|---|---|---|
| Tumor | Treatment | Tumor node volume at the day 21 after tumor inoculation (mm³) | Inhibition of tumor growth (%) |
| | | | |

| (a) Melanoma B16 | | | |
|---|---|---|---|
| | Control (PBS p.o.) | 765±69 | - |
| | Control (PBS i.v.) | 736±51 | - |
| | C₇H₁₅MDP p.o. | 571±65 | 25 |
| | C₇H₁₅MDP i.v. | 494±49 | 33 |

| (b) Lymphoma EL-4 | | | |
|---|---|---|---|
| | Control (PBS p.o.) | 842±78 | - |
| | Control (PBS i.v.) | 789±64 | - |
| | C₇H₁₅MDP p.o. | 650±73 | 23 |
| | C₇H₁₅MDP i.v. | 463±52 | 41 |

Thus, the preparation has significant antitumor activities, which have been demonstrated by prolongation of life-span of tumor-bearing animals (table 6) and inhibition of tumor growth (table 7). Further trials of the preparation have shown that the treatment, when begun 1 week before tumor implantation, was more successful, and complete regression of tumor was observed in several cases. The treatment with liposomal immunomodulator has been proved to be more effective compared to unencapsulated preparation. However, all methods of administration (p.o., i.v., liposomal i.v.) seem to be rational. Exact administration method is to be defined individually taking into account localization and type of tumor and other factors.

Wide studies of β-heptylglycoside-MDP (C₇H₁₅MDP) as immunomodulator made it possible to pass from experimental investigations to clinical trials. For this purpose, pharmaceutical compositions for p.o. and i.v. administration including liposomal form have been created.

Pharmaceutical composition for p.o. administration is a solid form of drug that includes the active ingredient in dose of 0.0001-100 mg/kg in weight and physiologically acceptable carriers.

Pharmaceutical composition for i.v. administration including liposomal form contains active ingredient in dose of 0.00001-30 mg/kg and 0.00001-10 mg/kg in weight, respectively.

Treating and prophylactic activities of food additive are based on the fact that the active substance stimulates the main components of the immunity including antitumor immune response: helper and cytotoxic T-lymphocytes, macrophages, natural killers, B-lymphocytes. Stimulation of the above-mentioned cell populations is a mechanism of C₇H₁₅MDP biological and in particular antitumor activities in the human body.

For the first time, possibility and expediency of C₇H₁₅MDP application as a food additive, which is introduced into food (mainly liquid food) have been established. Positive aspects of such application are discussed bellow.

Natural unmodified muramyl dipeptide (N-acetylmuramyl-L-alanyl-D-isoglutamine, abbreviation - MDP) is a structural component of cell wall peptidoglycan of some bacteria (Mycobacterium bovis, Streptococcus pyogenes, Corynebacterium parvum, etc.). After degradation of cell walls of these bacteria, glycopeptides that include in their structure MDP appear.

Muramyl peptides are constantly present in the human and animal bodies in low concentrations and play the role of sleep factors, vitamins and immunomodulators. As it was already mentioned, there are several serious obstacles in use of MDP for stimulation of the immune reactions in vivo. Due to high hydrophilicity, after systemic administration it is rapidly cleared from the circulation and excreted with urine, and therefore it failed to stimulate the functions of immune cells. Moreover, it has a significant concomitant pyrogenic effect.

Perspective immunomodulator amphiphilic preparation CH₃ -(CH₂)n -MDP, which is proposed as an active compound of food additive, is not a component of bacterial cell wall. It is synthetic derivative of natural bacterial muramyl dipeptide.

The preparation is a more effective stimulator of the main components of the immunity compared to unmodified MDP and its more lipophilic homologues. Evidently, its is caused by advantages of amphiphilic preparation in penetration through biological membranes and peculiarities of its intracellular traffic. Positive results of investigations of CH₃ -(CH₂)n -MDP as a stimulator of antitumor response in model systems in vivo and its low toxicity point to the expediency of its application.

Amphiphilicity is an important character of MDP derivatives, which allows to penetrate effectively through biological membranes and influence processes in cytoplasma leading to lymphocyte and macrophage activation. Amphiphilic preparation has advantages in comparison with intensively studied lipophilic derivatives (MTP-PE, MDP-GDP, MTP-Chol) in the sense, that it is characterized by better solubility in aqueous solutions, which significantly expends the sphere of the preparation use.

CH₃ -(CH₂)n -MDP is a powder of white or slightly gray color, which is soluble in water after 1 minute mixing at room temperature. It is expedient to introduce it into liquid food (juices, soups, etc.) or to use in gelatin capsules with physiologically acceptable carriers such as maltadextrine or food starch. The substance is characterized by good solubility and does not influence significantly organoleptic, chemical and physical properties of the food. It does not take part in chemical reactions with food components. The substance establishes only hydrophilic-hydrophobic bonds with food components, which dissociate easily in process of digestion and absorption.

Dose of the active ingredient of food additive is of 0.0001-100 mg/kg in weight.

E.g.: Man of 70-kg weight takes food additive that includes the active ingredient in dose of 0,007-7000 mg.

The main advantage of application of CH₃ -(CH₂)n -MDP as a food additive is lower toxicity and pyrogenicity, which may be observed in some cases of the preparation use in form of drug.

Decrease of toxicity and pyrogenicity is due to slowed absorption of the substance, when administered with food, in digestive tract. The substance enters the body gradually. When to preparation is administered per os in form of drug, the short-term rapid increase of the preparation concentration in the body tissues, which is followed by relatively quick decrease, is observed. During the period of the peak concentration the substance stimulate redundantly the functions of monocytes/macrophages, which release rapidly tumor necrosis factor, interleukin-1 and some other biologically active factors causing toxic and pyrogenic effects. If CH₃-(CH₂)n-MDP is administered in form of food additive, there is no such rapid increase of the preparation concentration in the body tissues. Moreover, delayed absorption leads to more prolonged entering of the substance into the body. As a result, rather long period, during which the preparation concentration in tissues is optimal for physiological stimulation of immune cells, is observed without rapid increase and decrease. Thus, a new application of the known substance has been proposed.

## Claims

1. The use of α- and/or β-glycosides of N-acetylmuramyl-L-alanyl-D-isoglutamine with mutual chemical structure: 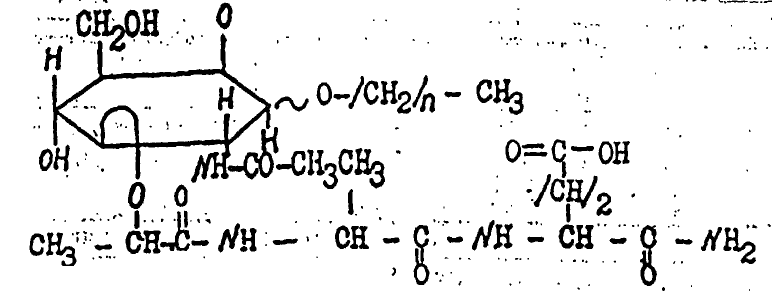 as an immunomodulator.

2. Pharmaceutical composition with antitumor properties that includes an effective quantity of α- and/or β-glycosides of N-acetylmuramyl-L-alanyl-D-isoglutamine /CH₃-(CH₂)n-MDP/ as an active ingredient and physiologically acceptable carriers.

3. Pharmaceutical composition mentioned in point 2, which is destined for intravenous, subcutaneous and intramuscular administration and includes the active ingredient in dose of 0.00001-30 mg/kg in weight.

4. Pharmaceutical composition mentioned in point 2 or 3, which is prepared in liposomal form and includes the active ingredient in dose of 0.00001-10 mg/kg in weight.

5. Pharmaceutical composition mentioned in point 2, which is destined for peroral administration and includes the active ingredient in dose of 0.0001-100 mg/kg in weight.

6. The use of α- and/or β-glycosides of N-acetylmuramyl-L-alanyl-D-isoglutamine as a food additive.
